# EUROPÄISCHE PATENTSCHRIFT

(11) **EP 0 974 246 B1**
(45) Veröffentlichungstag und Bekanntmachung des Hinweises auf die Patenterteilung: **15.01.2003**
(21) Anmeldenummer: 98919077.2
(22) Anmeldetag: 28.03.1998
(51) Int. Cl.: H05B 37/03, H05B 41/295

(54) **VERFAHREN UND SCHALTUNGSANORDNUNG ZUM BETREIBEN EINES ELEKTRISCHEN LEUCHTMITTELS**
METHOD AND CIRCUIT FOR OPERATING AN ELECTRICAL LIGHT
PROCEDE ET CIRCUIT POUR FAIRE FONCTIONNER UN ELEMENT ELECTRIQUE LUMINEUX

(30) Priorität: 08.04.1997 DE 19714416
(43) Veröffentlichungstag der Anmeldung: 26.01.2000
(73) Patentinhaber: KBL Solarien AG, 56307 Dernbach (DE)
(72) Erfinder: BECKER, Norbert, D-56581 Neuwied (DE)
(74) Vertreter: Beyer, Rudi
(86) Internationale Anmeldenummer: DE9800903
(87) Internationale Veröffentlichungsnummer: WO98046049

(56) Entgegenhaltungen:
- EP-A- 0 422 255
- WO-A-93/21568
- DE-A- 2 554 889
- DE-A- 4 426 664
- US-A- 4 831 564

## Beschreibung

Die Erfindung betrifft ein Verfahren zum Betreiben einer oder mehrerer UV-Niederdrucklampen, zur Verwendung in Solarien.

Des weiteren betrifft die Erfindung ein Solarium mit einer oder mehreren UV-Niederdrucklampen, einer elektrischen Energiequelle und einem Vorschaltgerät.

Es ist bekannt, daß die Strahlungsleistung von bei der künstlichen Besonnung eingesetzten UV-Niederdrucklampen bereits nach relativ kurzer Betriebsdauer (Gesamt-Betriebsdauer) soweit abnimmt, daß diese durch eine neue UV-Niederdrucklampe ersetzt werden muß. Anderenfalls wären inakzeptabel lange - und stetig länger werdende - Benutzungszeiten im Hinblick auf ein Bestrahlungsergebnis bei der Besonnung die unvermeidliche Folge. Der notwendige Austausch der UV-Niederdrucklampen verursacht allerdings beträchtliche Kosten. Darüber hinaus sind die verbrauchten UV-Niederdrucklampen als Sondermüll zu entsorgen.

Um die Betriebsdauer der relativ teuren UV-Niederdrucklampen von üblicherweise etwa 500 Stunden zu verlängern, hat man auch schon versucht, Eingriffe im Bereich der bei diesen Gasentladungslampen notwendigen elektrischen oder elektronischen Vorschaltgeräte vorzunehmen, derart, daß manuell eine Umschaltung der an die Gasentladungslampen abgegebenen elektrischen Leistung vorgenommen wird in dem Sinne, daß im Fall einer bereits mehr oder weniger stark abgefallenen Strahlungsleistung der betreffenden UV-Niederdrucklampen die zuzuführende elektrische Energie sprunghaft um einen größeren Betrag erhöht wird, so daß wieder eine Strahlungsleistung der betreffenden UV-Niederdrucklampe erreicht wird, die mehr oder weniger genau derjenigen zu Beginn der Inbetriebnahme entspricht.

Aus der US-A-4 831 564 ist es bekannt, anhand neuer, ungenutzter Xenon-Lampen zu analysieren, wie die erhöhte Entladungsleistung mit der Zeit bei konstanter Lichtbestrahlung variiert. Dieses Verfahren wird bei mehreren unbenutzten Lampen durchgeführt, und die Ergebnisse werden gespeichert. Die der Xenon-Lampe zuzuführende elektrische Entladungsleistung wird automatisch kontrolliert, damit die Strahlungsleistung konstant bleibt.

Der Erfindung liegt die Aufgabe zugrunde, auf möglichst einfache und dabei kostengünstige Weise die gesamte Nutzungsdauer (Gesamt-Betriebsdauer) einer UV-Niederdrucklampe in Solarien erheblich zu verlängern, ohne daß dabei den praktischen Nutzungsbetrieb von Solarien störende Faktoren, zum Beispiel übermäßig lang werdende und/oder mehr oder weniger stark variierende Besonnungszeiten, die Folge sind.

Diese Aufgabe wird durch die in **Patentanspruch 1** wiedergegebenen Merkmale gelöst.

Die Anwendung des erfindungsgemäßen Verfahrens ermöglicht ein Betreiben von Solarien über einen im Vergleich zur bisherigen bekannten Nutzungsweise erheblich längeren Zeitraum hinweg, und zwar bei zumindest im wesentlichen gleichbleibender Strahlungsleistung, die individuell vorgegeben wurde. Infolgedessen können zum Beispiel gerade beim Betrieb von Solarien erhebliche Kosten durch bessere Ausnutzung der üblicherweise verwendeten UV-Niederdrucklampen eingespart werden, ohne daß sich dies durch mehr oder weniger stark variierende Besonnungszeiten nachteilig bemerkbar macht.

Bezüglich der artspezifischen Daten einer UV-Niederdrucklampe wird ausgeführt, daß hiermit insbesondere die für die betreffende UV-Niederdrucklampe beispielsweise aus Herstellerangaben bekannte Abnahme der Strahlungsleistung in Abhängigkeit von der Gesamt-Betriebsdauer des individuellen Leuchtmittels gemeint ist. Aber auch andere Abhängigkeiten des Lichtabgabe- bzw. Strahlungsverhaltens der betreffenden UV-Niederdrucklampen können hierzu gehören, wie beispielsweise die Temperatur der Umgebung, in der das Leuchtmittel betrieben wird. Im Falle der erwähnten UV-Niederdrucklampe sind ferner die elektrischen oder elektronischen Daten des jeweils eingesetzten elektrischen oder elektronischen Vorschaltgerätes von Interesse und können in den Steuerungsvorgang bezüglich der Strahlungsabgabe der betreffenden UV-Niederdrucklampe einbezogen werden.

Weitere vorteilhafte Ausgestaltungen der Erfindung sind in den **Patentansprüchen 2** bis **8** bezüglich des Verfahrens beschrieben.

Hinsichtlich der Lösung für das Solarium beschreibt **Patentanspruch 9** die erfinderische Lösung.

Ein derartiges Solarium läßt sich kostengünstig betreiben und produziert wenig Sondermüll.

Weitere vorteilhafte Ausgestaltungen sind in den **Patentansprüchen 10** bis **12** beschrieben.

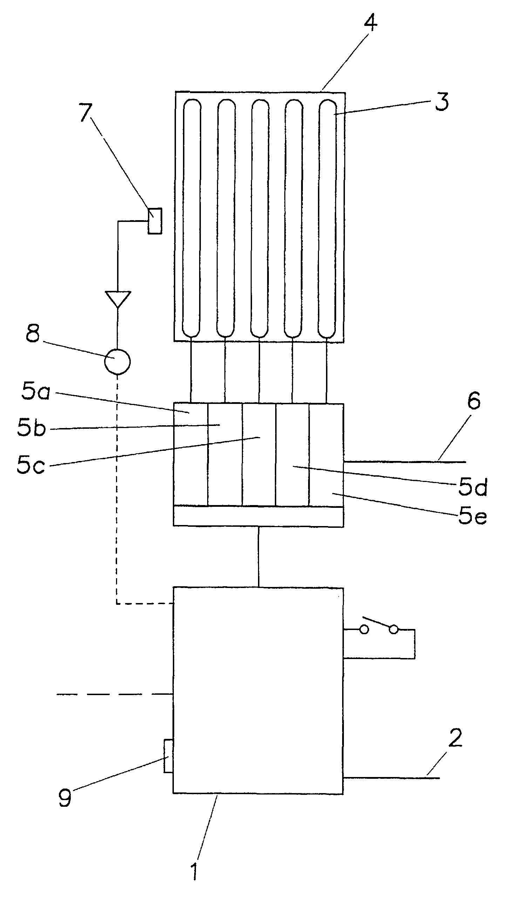

Die Erfindung wird nachfolgend anhand eines in der Zeichnung dargestellten Ausführungsbeispieles näher erläutert.

In dieser Zeichnung ist als sogenanntes Blockdiagramm ein Steuerkreis gezeichnet, der zunächst aus einem als Prozessor ausgebildeten Steuergerät 1 besteht. Dieses Steuergerät 1 wird über eine Leitung 2 mit einer üblichen Spannung, beispielsweise 220 V, versorgt. Im dem Steuergerät 1 sind mehrere beispielsweise zehn Kompensationskennlinien von marktüblichen Gasentladungslampen 3 gespeichert, die in einem UV-Bestrahlungsgerät 4 beispielsweise für den menschlichen Körper bzw. Teile davon eingesetzt sind.

Derartige UV-Lampen 3 haben die Eigenart, daß mit zunehmender Brenndauer die sogenannte UV-Ausgangsleistung verhältnismäßig stark abnimmt. Beispielsweise nach einer Brenndauer von etwa 500 Stunden verringert sich die UV-Ausgangsleistung um etwa 30%. Diese Reduzierung ist unbefriedigend und führt, obwohl die UV-Lampen 3 eigentlich noch brauchbar sind, zu einem Austausch und damit zu einer Produktion von Sondermüll. Darüber hinaus sind derartige UV-Lampen 3 verhältnismäßig teuer.

Zwischen dem UV-Bestrahlungsgerät 4 bzw. dessen UV-Lampen 3 sind nun mehrere, zu einem Block zusammengefaßte, elektronische Vorschaltgeräte 5a - 5e angeordnet, von denen hier jeweils ein Vorschaltgeräte 5a bis 5e einer UV-Lampe 3 zugeordnet ist. Es ist auch möglich, daß ein Vorschaltgerät 5a vorgesehen ist, welches zwei oder mehr UV-Lampen 3 versorgt. Die Vorschaltgeräte 5a - 5e sind an eine Stromversorgungsleitung angeschlossen. Die elektronischen Vorschaltgeräte 5a - 5e werden nun, in Abhängigkeit von gleichen oder unterschiedlichen Typen der UV-Lampen 3, über eine oder mehrere der im Steuergerät 1 gespeicherten und selektierten Kompensationskennlinien so gesteuert, daß den einzelnen oder ausgewählten UV-Lampen 3 immer eine solche, jedoch zunehmende Stromstärke für die Versorgung der UV-Lampen 3 zugeführt wird, daß dieselben immer eine gleichbleibende UV-Ausgangsleistung abgeben. Diese annähernd konstante Ausgangsleistung kann entweder bei der vollen Leistung der UV-Lampen 3 oder, bei einer in gewissen Grenzen reduzierten Leistung liegen, wobei sich im letzteren Fall die Brenndauer der UV-Lampen 3 erhöht. Dadurch ist es beispielsweise möglich, UV-Lampen 3 mit einer höheren normalerweise nicht zulässigen UV-Strahlungsintensität zu verwenden, die nur eine reduzierte, jedoch weitgehend konstante UV-Leistung abgeben.

Die von einer oder mehreren Gasentladungslampen 3 zu einem beliebigen Zeitpunkt tatsächlich abgegebene Licht- bzw. Strahlungsleistung kann über mindestens einen Sensor 7 gemessen und mit einem in einer Einheit 8 gespeicherten Sollwert verglichen werden. Bei Abweichungen vom Sollwert wird dies dem Steuergerät 1 übermittelt, welches dann die elektronischen Vorschaltgeräte 5a bis 5e entsprechend beeinflußt.

Es ist möglich, die Temperatur im Bereich der Gasentladungslampen und/oder der Umluft, die die Arbeit der Gasentladungslampen nachteilig beeinträchtigt, zu messen und als Korrekturgröße dem Steuergerät 1 zuzuführen.

Die den Vorschaltgeräten 5a - 5e vom Steuergerät 1 zugeführten Steuersignale können sowohl digital als auch analog sowie strom-, spannungs-, oder frequenzmoduliert sein. Dem Steuergerät 1 ist ein sogenannter Reset-Schalter 9 zugeordnet, der nach dem Auswechseln der Gasentladungslampen 3 für einen Neustart wird.

## Patentansprüche

1. Verfahren zum Betreiben einer oder mehrerer UV-Niederdrucklampen (3), zur Verwendung in Solarien, wobei für die jeweils benutzte/benutzten UV-Niederdrucklampe(n) arttypische Daten bezüglich Strahlenintensität und deren Abnahme in Abhängigkeit der Gesamt-Betriebsdauer bestimmt und in einem elektronischen Speicher abgelegt und die jeweiligen Einzelbetriebszeiten der betreffenden UV-Niederdrucklampe (3) gemessen und jeweils zu den vorangegangenen Einzelbetriebszeiten addiert und die so bestimmten Summen der Einzelbetriebszeiten jeweils gespeichert werden, wobei die der betreffenden UV-Niederdrucklampe (3) zu einem Zeitpunkt zugeführte elektrische Energie in Abhängigkeit von den abgespeicherten arttypischen Daten und in Abhängigkeit von der jeweils gespeicherten Summe der Einzelbetriebszeiten zwecks gleichbleibender oder annähernd gleichbleibender Strahlungsleistung der betreffenden UV-Niederdrucklampe (3) selbsttätig erhöht wird.

2. Verfahren nach Anspruch 1, **dadurch gekennzeichnet, daß** zusätzlich zu den arttypischen Daten der betreffenden UV-Niederdrucklampe (3) auch relevante Daten eines Vorschaltgerätes (5d, 5e) in dem elektronischen Speicher abgespeichert werden.

3. Verfahren nach Anspruch 1, **dadurch gekennzeichnet, daß** die der betreffenden UV-Niederdrucklampe (3) zuzuführende elektrische Energie so eingestellt wird, daß sie um einen gewissen Bruchteil, beispielsweise um etwa zehn Prozent, geringer ist als die elektrische Nennleistung der betreffenden UV-Niederdrucklampe (3).

4. Verfahren nach Anspruch 2 oder 3, **dadurch gekennzeichnet, daß** dem Vorschaltgerät (5d, 5e) die zum Einstellen der jeweiligen an die UV-Niederdrucklampe (3) abzugebende elektrische Energie erforderlichen Steuersignale in digitaler Form zugeführt werden.

5. Verfahren nach Anspruch 2 oder 3, **dadurch gekennzeichnet, daß** dem Vorschaltgerät (5d, 5e) die zum Einstellen der jeweiligen an die UV-Niederdrucklampe (3) abzugebende elektrische Energie erforderlichen Steuersignale in analoger Form zugeführt werden.

6. Verfahren nach Anspruch 3 oder 4, **dadurch gekennzeichnet, daß** die Steuersignale strommoduliert sind.

7. Verfahren nach Anspruch 3 oder 4, **dadurch gekennzeichnet, daß** die Steuersignale spannungsmoduliert sind.

8. Verfahren nach Anspruch 3 oder 4, **dadurch gekennzeichnet, daß** die Steuersignale frequenzmoduliert sind.

9. Solarium mit einer oder mehreren UV-Niederdrucklampen, einer elektrischen Energiequelle und einem Vorschaltgerät, **gekennzeichnet durch** im Stromkreis der betreffenden UV-Niederdrucklampe (3) liegende elektrische oder elektronische, einen Speicher für arttypische Daten der betreffenden UV-Niederdrucklampe (3) enthaltende Steuermittel zum Einstellen einer der betreffenden UV-Niederdrucklampe (3) zwecks Erhalt einer vorgegebenen, wenigstens annähernd konstant bleibenden Strahlungsleistung der UV-Niederdrucklampe (3) zuzuführenden elektrischen Leistung sowie mit einem der UV-Niederdrucklampe (3) zugeordneten Zeitmeßmittel, das die jeweilige Betriebsdauer der zugeordneten UV-Niederdrucklampe (3) mißt, mit einem Speicher, in dem die jeweils gemessene Betriebsdauer der zugeordneten UV-Niederdrucklampe (3) zu der Gesamt-Betriebsdauer der UV-Niederdrucklampe (3) addierbar ist und diese Gesamt-Betriebsdauer jeweils in dem Speicher abspeicherbar ist, mit einem Vorschaltgerät (5a bis 5e), das Steuersignale an die UV-Niederdrucklampen (3) zwecks Einhaltung einer annähernd konstanten Strahlungsleistung der UV-Niederdrucklampe (3) **durch** Vergleich der Gesamt-Betriebsdauer mit den abgespeicherten artspezifischen Daten an die UV-Niederdrucklampe (3) abgibt.

10. Solarium nach Anspruch 9, **dadurch gekennzeichnet, daß** die Einstellmittel zur Initialisierung der Zeitmessung für die betreffende UV-Niederdrucklampe (3) von Hand betätigbar sind.

11. Solarium nach Anspruch 10, **dadurch gekennzeichnet, daß** die Einstellmittel zur Initialisierung der Zeitmessung benachbart zu der in einer Fassung oder dergleichen befestigten UV-Niederdrucklampe (3) angeordnet sind und zusammen mit einer Bewegung der UV-Niederdrucklampe (3) in die Fassung bzw. aus der Fassung oder dergleichen betätigbar sind.

12. Solarium nach Anspruch 9 oder einem der darauffolgenden Ansprüche, **dadurch gekennzeichnet, daß** die Steuermittel zum Einstellen der der UV-Niederdrucklampe (3) zuzuführenden elektrischen Leistung und/oder die Zeitmeßmittel mit einer optischen und/oder akustischen Signaleinrichtung verbunden sind zum Signalisieren der erreichten, vorgegebenen Gesamt-Betriebsdauer der UV-Niederdrucklampe (3).

## Claims

1. A method for operating one or several UV low-pressure lamps (3) for use in solaria, with type-representative data regarding the radiation intensity and its reduction for the UV low-pressure lamp(s) used each time being determined as a function of the total operation time and stored in an electronic memory, and the respective individual operating times of the relevant UV low-pressure lamp (3) being measured and in each case added to the preceding operating times, and the sums of the individual operating times thus determined being stored in each case, the electrical energy being supplied to the relevant UV low-pressure lamp (3) at a point in time as a function of the stored type-representative data and being increased automatically as a function of the sum of the individual operating times stored in each case for the purpose of constant or roughly constant radiation power of the relevant UV low-pressure lamp (3).

2. A method as claimed in Claim 1, **characterized in that,** in addition to the type-representative data of the relevant UV low-pressure lamp (3), relevant data of a ballast unit (5d, 5e) are also being stored in the electronic memory.

3. A method as claimed in Claim 1, **characterized in that** the electrical energy to be supplied to the relevant UV low-pressure lamp (3) is set in such a way that it is lower by a certain fraction, for instance by some ten per cent, than the electrical rated power of the relevant UV low-pressure lamp (3).

4. A method as claimed in Claim 2 or Claim 3, **characterized in that the** control signals required for setting the respective electrical energy to be supplied to the UV low-pressure lamp (3) are applied in digital form.

5. A method as claimed in Claim 2 or Claim 3, **characterized in that the** control signals required for setting the respective electrical energy to be supplied to the UV low-pressure lamp (3) are applied in analogue form.

6. A method as claimed in Claim 3 or Claim 4, **characterized in that** the control signals are current modulated.

7. A method as claimed in Claim 3 or Claim 4, **characterized in that** the control signals are voltage modulated.

8. A method as claimed in Claim 3 or Claim 4, **characterized in that** the control signals are frequency modulated.

9. A Solarium with one or several UV low-pressure lamps, an electric energy source and a ballast unit, **characterized by** electric or electronic control means lying in the circuit of the relevant UV low-pressure lamp (3) and comprising a memory for type-representative data of the relevant UV low-pressure lamp (3) for setting the electrical power to be supplied to the UV low-pressure lamp (3) for the purpose of maintaining a specified, at least roughly constant, radiation power of the relevant UV low-pressure lamp (3), as well as with a time-interval measuring means associated with the UV low-pressure lamp (3) which measures the respective operating time of the associated UV low-pressure lamp (3); with a memory in which the operating time of the associated UV low-pressure lamp (3) measured in each case can be added to the total operating time of the UV low-pressure lamp (3) and this total operating time being storable in the memory in each case; with a ballast unit (5a to 5e) which gives control signals to the UV low-pressure lamp (3) for the purpose of maintaining a roughly constant radiation power of the UV low-pressure lamp (3) by comparing the total operating time with the stored, type-representative data of the UV low-pressure lamp (3).

10. A Solarium as claimed in Claim 9, **characterized in that the** setting means for initialisation of the time measurement for the relevant UV low-pressure lamp (3) can be operated manually.

11. A Solarium as claimed in Claim 10, **characterized in that the** setting means for initialisation of the time measurement are arranged next to the UV low-pressure lamp (3) fixed in a socket or the like and are operable together with a movement of the UV low-pressure lamp (3) into the socket and/or out of the socket or the like.

12. A solarium as claimed in Claim 9 or in any of the following claims, **characterized in that** the control means for setting the electric power to be supplied to the UV low-pressure lamp (3) and/or the time-interval measuring means are connected to a visual or acoustic signalling device for signalling the reached, specified total operating time of the UV low-pressure lamp (3).

## Revendications

1. Procédé d'actionnement d'une ou de plusieurs lampes UV basse pression (3) destinées à être utilisées dans des solariums, les données typiques de la (des) lampe(s) UV basse pression utilisée(s) concernant l'intensité de rayonnement et la réception correspondante en fonction de la durée de service globale étant déterminées et enregistrées dans une mémoire électronique, les durées de service individuelles respectives de la lampe UV basse pression correspondante (3) étant mesurées et ajoutées respectivsment aux durées de service individuelles précédentes, les sommes ainsi déterminées des durées de service individuelles étant respectivement enregistrées, l'énergie électrique transférée à un moment donné à la lampe UV basse pression correspondante (3) étant automatiquement accrue en fonction des données typiques enregistrées et en fonction de la somme respectivement enregistrée des durées de service individuelles en vue d'assurer une puissance de rayonnement constante ou pratiquement constante de la lampe UV basse pression respective (3).

2. Procédé selon la revendication 1, **caractérisé en ce que** des données importantes d'un appareil monté en série (5d, 5e) sont aussi enregistrées dans une mémoire électronique, en plus des données typiques de la lampe UV basse pression respective (3).

3. Procédé selon la revendication 1, **caractérisé en ce que** l'énergie électrique transférée à la lampe UV basse pression respective (3) est réglée de sorte à être inférieure d'une certaine fraction, par exemple d'environ dix pour cent, à la puissance électrique nominale de la lampe UV basse pression respective (3).

4. Procédé selon les revendications 2 ou 3, **caractérisé en ce que** les signaux de commande nécessaires au réglage de la lampe UV basse pression respective (3) sont transmis à l'appareil monté en série (5d, 5e) sous une forme numérique.

5. Procédé selon les revendications 2 ou 3, **caractérisé en ce que** les signaux de commande nécessaires au réglage de la lampe UV basse pression respective (3) sont transmis à l'appareil monté en série (5d, 5e) sous une forme analogique.

6. Procédé selon les revendications 3 ou 4, **caractérisé en ce que** les signaux de commande sont modulés en intensité.

7. Procédé selon les revendications 3 ou 4, **caractérisé en ce que** les signaux de commande sont modulés en tension.

8. Procédé selon les revendications 3 ou 4, **caractérisé en ce que** les signaux de commande sont modulés en fréquence.

9. Solarium comportant une ou plusieurs lampes UV basse pression, une source d'énergie électrique et un appareil monté en série, **caractérisé par** des moyens de commande électriques ou électroniques agencés dans le circuit de la lampe UV basse pression respective (3), contenant une mémoire pour les données typiques de la lampe UV basse pression respective (3), servant au réglage de l'énergie électrique devant être transférée à la lampe UV basse pression respective (3) en vue d'assurer une puissance de rayonnement prédéterminée au moins pratiquement constante de la lampe UV basse pression respective (3), et comportant un moyen de mesure du temps affecté à la lampe UV basse pression (3), mesurant la durée de service de la lampe UV basse pression affectée (3), et une mémoire, dans laquelle la durée de service respectivement mesurée de la lampe UV basse pression affectée (3) peut être additionnée à la durée de service globale de la lampe UV basse pression (3), cette durée de service globale pouvant respectivement être enregistrée dans la mémoire, un appareil monté en série (5a à 5e), transmettant des signaux de commande aux lampes UV basse pression (3) en vue de maintenir une puissance de rayonnement pratiquement constante de la lampe UV basse pression (3), par comparaison de la durée de service globale avec les données typiques enregistrées de la lampe UV basse pression (3).

10. Solarium selon la revendication 9, **caractérisé en ce que** les moyens de réglage servant à l'initialisation de la mesure du temps de la lampe UV basse pression respective (3) peuvent être actionnés manuellement.

11. Solarium selon la revendication 10, **caractérisé en ce que** les moyens de réglage servant à l'initialisation de la mesure du temps sont agencés au voisinage de la lampe UV basse pression (3) fixée dans une douille ou un élément similaire et peuvent être actionnés simultanément à un déplacement de la lampe UV basse pression (3) dans la douille ou hors de celle-ci.

12. Solarium selon la revendication 9 ou selon l'une des revendications suivantes, **caractérisé en ce que** les moyens de commande servant au réglage de l'énergie électrique devant être transférée à la lampe UV basse pression (3) et/ou les moyens de mesure du temps sont reliés à un dispositif de signalisation optique et/ou acoustique en vue de signaler la durée de service globale prédéterminée atteinte de la lampe UV basse pression (3).
